# EUROPÄISCHE PATENTSCHRIFT

(11) **EP 2 097 366 B1**
(45) Veröffentlichungstag und Bekanntmachung des Hinweises auf die Patenterteilung: **10.11.2010**
(21) Anmeldenummer: 00993318.5
(22) Anmeldetag: 27.11.2000
(51) Int. Cl.: C07C 51/60, C07C 63/04, C07C 63/10

(54) **VERFAHREN ZUR HERSTELLUNG VON O-CHLORMETHYLBENZOESÄURECHLORIDEN**
METHOD FOR PRODUCING O-CHLOROMETHYL BENZENECARBONYL CHLORIDES
PROCEDE DE FABRICATION DE CHLORURES D'ACIDE O-CHLOROMETHYL-BENZOIQUE

(30) Priorität: 06.12.1999 DE 19958601
(43) Veröffentlichungstag der Anmeldung: 09.09.2009
(73) Patentinhaber: BASF SE, 67056 Ludwigshafen (DE)
(72) Erfinder: STAMM, Armin, 55128 Mainz (DE); GÖTZ, Roland, 68809 Neulussheim (DE); GÖTZ, Norbert, 67547 Worms (DE); HENKELMANN, Jochem, 68165 Mannheim (DE); KNEUPER, Heinz-Josef, 67150 Niederkirchen (DE); WOLF, Bernd, 67136 Fussgönheim (DE)
(86) Internationale Anmeldenummer: PCT/EP2000/011814
(87) Internationale Veröffentlichungsnummer: WO 2001/042183

(56) Entgegenhaltungen:
- EP-A- 0 583 589
- WO-A-97/12854
- WO-A-99/16743

## Beschreibung

Die vorliegende Erfindung betrifft ein Verfahren zur Herstellung von o-Chlormethylbenzoesäurechloriden der Formel I, 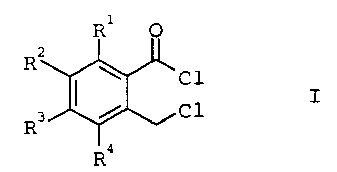 in der R¹ bis R⁴ gleich oder verschieden sein können und für Wasserstoff, C₁-C₄-Alkyl, Halogen oder Trifluormethyl stehen, durch Umsetzung von benzokondensierten Lactonen der Formel II, 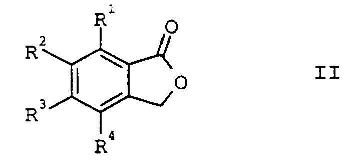 in der R¹ bis R⁴ die vorgenannte Bedeutung besitzen, mit gasförmigem oder flüssigem Phosgen, dessen Dimeren oder Trimeren.

o-Chlormethyl-substituierte Benzoylchloride sind wichtige Zwischenprodukte zur Herstellung von beispielsweise pestiziden Wirkstoffen wie sie in den Patentschriften EP-A 460 575, EP-A 463 488, WO-A 95/18789, WO-A 95/21154 und WO-A 97/15552 beschrieben sind.

o-Chlormethyl-substituierte Benzoylchloride können zum Beispiel durch Umsetzung von benzokondensierten Lactonen mit Thionylchlorid oder Phosgen hergestellt werden.

In EP-A 676 389 wird die Herstellung von o-Chlormethylbenzoesäurechloriden aus benzokondensierten Lactonen mit Thionylchlorid in Gegenwart eines Katalysators beschrieben. Um einen vollständigen Umsatz zu erzielen, sind Reaktionstemperaturen von 160 bis 170°C erforderlich, bei denen sich Thionylchlorid bereits teilweise zersetzt und infolgedesssen störende Nebenprodukte gebildet werden.

In der WO-A 99/16743 wird die Umsetzung mit Thionylchlorid in Gegenwart eines quartären Ammoniumsalzes und einer Lewis-Säure bei 90 und 100°C durchgeführt. Quartäre Ammoniumsalze sind jedoch unter Umweltaspekten problematisch und haben folgende verfahrenstechnische Nachteile: durch Sublimation kann es zur Verstopfung von Anlagenteile kommen. Weiterhin sind die Salze hygroskopisch, was zum Wassereintrag und einem Mehrverbrauch am Chlorierungsmittel führen kann. Schließlich stören die Ammoniumsalze bei der destillativen Aufreinigung der o-Chlormethylbenzoesäurechloride.

Die Reaktionsführung mit Thionylchlorid als chlorierendem Reagenz ist insofern nachteilig, da als Koppelprodukt der Synthese Schwefeldioxid gebildet wird, welches aufgearbeitet oder neutralisiert werden muß. Bei der Verwendung von Phosgen als Chlorierungsmittel fällt lediglich Kohlendioxid an, welches nicht entsorgt werden muß.

EP-A 583 589 beschreibt ein Verfahren zur Herstellung von o-Chlormethylbenzoesäurechloriden durch Phosgenierung von benzokondensierten Lactonen in Gegenwart eines Katalysators bei 170 bis 180°C. Zwar ist Phosgen unter diesen Bedingungen im Gegensatz zu Thionylchlorid thermisch stabil, jedoch wird die Handhabung von Phosgen und dessen Holdup im Kühler bei den hohen Temperaturen durch einen erhöhten sicherheitstechnischen Aufwand erschwert. Des weiteren wird das Reaktionsprodukt unter diesen Bedingungen thermisch stark belastet, was zu seiner teilweisen Zersetzung führen kann.

In WO 97/12854 werden als spezieller Katalysatortyp Triarylphosphinoxide für die Reaktion verwendet, wodurch auf die Verwendung von Chlorwasserstoff verzichtet werden kann, was jedoch zu keiner Verbesserung der erforderlichen Reaktionsbedingungen hinsichtlich der bedenklich hohen Reaktionstemperaturen und dem damit notwendigen sicherheitstechnischen Aufwand führt.

Aufgabe der vorliegenden Erfindung war es daher ein wirtschaftliches und prozeßfähiges Verfahren zur Herstellung von o-Chlormethylbenzoesäurechloriden zu finden, das die oben aufgeführten Nachteile nicht aufweist und dennoch hohe Ausbeuten liefert.

Demgemäß wurde das eingangs erwähnte Verfahren gefunden, das dadurch gekennzeichnet ist, daß die Umsetzung mit Phosgen, dessen Dimeren oder Trimeren in Gegenwart katalytischer Mengen einer Lewis-Säure und katalytischer Mengen eines Phosgenierungskatalysators erfolgt.

Zum Einsatz gelangen benzokondensierte Lactone (Phthalide) der allgemeinen Formel II 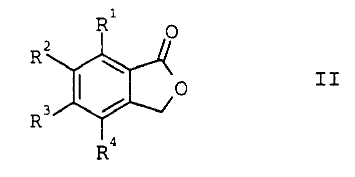 in der R¹ bis R⁴ gleich oder verschieden sein können und für Wasserstoff (H), C₁-C₄-Alkyl, Halogen (Fluor, Chlor, Brom oder Iod) oder Trifluormethyl stehen. Bevorzugt wird unsubstituiertes Phthalid eingesetzt.

Als Chlorierungsmittel wird vorzugsweise gasförmiges oder flüssiges Phosgen eingesetzt. Weiterhin können auch Dimere (Chlorameisensäuretrichlormethylester, "Diphosgen") oder Trimere des Phosgens (Kohlensäurebis(trichlormethyl)ester, "Triphosgen") oder Gemische dieser Chlorierungsmittel eingesetzt werden.

Als Phosgenierungskatalysator sind insbesondere Stickstoff- und Phosphorverbindungen geeignet. Beispielsweise sind N,N-disubstituierte Formamide, Hexaalkylguanidiniumsalze, Trialkyl- oder im Arylteil ggf. substituierte Triaryl-Phosphine, Trialkyl- oder im Arylteil ggf. substituierte Triaryl-Phosphinoxide, N-substituierte Imidazole sowie substituierte oder unsubstituierte Pyridine in Betracht zu ziehen. Insbesondere sind die Pyridine der Formel IIIa 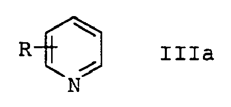 wobei R für Wasserstoff oder Methyl steht,
und Phosphinoxide der Formel IIIb, 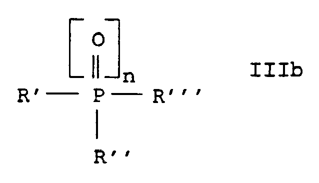 in der R' bis R"' gleich oder verschieden sein können und C₁-C₁₀-Alkyl oder unsubstituiertes oder durch C₁-C₄-Alkyl substituiertes Phenyl bedeuten und der Index n für 0 oder 1 steht, bevorzugt. Insbesondere bevorzugt ist 3-Methylpyridin (β-Picolin) und unsubstituiertes Triphenylphosphinoxid.

Der Einsatz von flüssigen Trialkylphosphinoxiden hat insbesondere verfahrenstechnische Vorteile (Vermeidung des Feststoffhandlings, einfacher Austrag des Destillationsrückstandes bei der Aufreinigung). Hier kommen beispielsweise die unter dem Handelsnamen Cyanex^{®} erhältlichen Tri-C₆-C₈-alkylphosphinoxide(s. z.B. Cyanex^{®} 923 der Fa. Cyanamid) in Betracht. Insbesondere haben sich die flüssigen Trialkylphosphinoxide in der Kombination mit Lewis-Säuren wie Borsäuretri(C₁-C₄-alkyl)ester und Borsäure bewährt.

Der Phosgenierungskatalysator wird in der Regel in Mengen von 0,1 bis 20 mol-% bezogen auf die eingesetzte Menge an benzokondensiertem Lacton, und bevorzugt in Mengen von 1 bis 10 mol-% zugesetzt.

Als Lewis-Säuren eignen sich insbesondere Borverbindungen, wie insbesondere BF₃, BCl₃ bzw. deren Komplexen mit Sauerstoff-, Schwefel- oder Stickstoffverbindungen, Borsäuretri(C₁-C₄-alkyl) -ester und die Borsäure (H₃BO₃) selbst. Desweiteren kommen AlCl₃, Alkylaluminiumdichloride und Dialkylaluminiumchloride sowie heterogene, Lewis-saure Aluminiumsilicate vom Zeolith-Typ in Frage. Besonders bevorzugt sind BF₃, BCl₃ und deren Komplexe mit Ether (insbesondere Diethylether), Wasser (Dihydrat), Alkohol (insbesondere Methanol), Sulfid (insbesondere Dimethylsulfid) und Amin (insbesondere Ethylamin). Insbesondere geeignet sind beispielsweise BF₃-Etherat und BF₃-Dihydrat.

Besonders bevorzugt werden Borsäure oder Borsäuretri(C₁-C₄-alkyl) -ester als Lewis-Säuren eingesetzt. Derartige Verfahren liefern hervorragende Ausbeuten und haben den Vorteil, daß die Reaktionsgemische frei von Fluoridionen sind. Damit vereinfacht sich im Vergleich zur analogen Reaktion mit BF₃ als Lewis-Säure die gesamte Apparatetechnik.

Die Lewis-Säure wird in Mengen von 0,1 bis 20 mol-% bezogen auf die eingesetzte Menge an benzokondensiertem Lacton, bevorzugt 0,5 bis 5 mol-%, zugegeben.

In einer weiteren bevorzugten Form des Verfahrens wird als Katalysator ein vorher gebildeter Komplex aus der Lewis-Säure und dem Phosgenierungskatalysator als Katalysatorsystem verwendet. In der Regel wird dieser Komplex in einer Konzentration von 0,1 bis 20 mol-% bezogen auf die eingesetzte Menge an benzokondensiertem Lacton eingesetzt. Bevorzugt wird ein Komplex aus BF₃ und einem Methyl-substituierten Pyridin als Katalysator verwendet, besonders bevorzugt ist der BF₃-β-Picolin-Komplex.

Optional kann parallel zur Phosgen-Einleitung Chlorwasserstoff zur Beschleunigung der Ringöffnung gasförmig eingeleitet werden. Bevorzugt wird jedoch auf die Einleitung von Chlorwasserstoff während der Synthese verzichtet.

Weiterhin kann es von Vorteil sein heterogene, Lewis-saure Katalysatoren wie z. B. Zeolithe vom Faujasit-Typ, bei denen austauschbare Kationen teilweise oder vollständig durch Protonen ersetzt sind, einzusetzen. Eine heterogen katalysierte Reaktion hat den Vorteil, daß sie im Festbett durchgeführt werden kann. Der heterogene Katalysator wird in Mengen von 0,01 bis 10 Gewichts-% und bevorzugt in Mengen von 0,1 bis 1 Gewichts-% bezogen auf die eingesetzte Menge an benzokondensiertem Lacton eingesetzt.

Die Reaktionstemperatur beträgt im allgemeinen 110 bis 200°C und vorzugsweise 130 bis 160°C.

Das Verfahren wird vorzugsweise ohne Lösungsmittel durchgeführt. Es ist jedoch möglich ein gegen Phosgen inertes Lösungsmittel zuzusetzen. Inerte Lösungsmittel sind z. B. aromatische Kohlenwasserstoffe wie Toluol, o-, m- oder p-Xylol oder deren Gemische, chlorierte aromatische Kohlenwasserstoffe wie Chlorbenzol oder Dichlorbenzolen oder cyclische Carbonate wie Ethylen- oder Propylencarbonat.

Das Verfahren kann sowohl kontinuierlich als auch diskontinuierlich durchgeführt werden.

Die nachfolgenden Beispiele sollen das Verfahren weiter erläutern.

Allgemeine Versuchsbeschreibung für alle Beispiele:
In einer Phosgenierungsapparatur bestehend aus einem 1 1 Doppelmantelreaktor mit aufgesetzter Intensivkühlungskaskade wurden jeweils 2 mol (268 g) Phthalid vorgelegt und zusammen mit dem jeweiligen Katalysatorsystem aufgeschmolzen. Nach Erwärmung auf 140 °C wurde solange Phosgen eingegast, bis kein Phosgen mehr verbraucht wird (erkennbar am Abfall der Innentemperatur). Danach ließ man eine Stunde bei 140°C nachrühren und strippte dann überschüssiges Phosgen mit Stickstoff bei 100°C. In ausgewählten Beispielen wurde das Produkt bei 1 mbar und 93-95°C durch fraktionierte Destillation isoliert.

### Beispiel 1

Als Katalysator wurden 9,3 g (0,1 mol, 5 mol-%) β-Picolin (3-Methylpyridin) und 7,9 g (0,04 mol, 2-mol-%) BF₃-Diethyletherkomplex ("BF₃-Etherat") zugegeben. Innerhalb von 8 h wurden bei 140-145°C insgesamt 218 g (2,18 mol) Phosgen eingegast. Der Reaktionsaustrag (387 g) enthielt nach dem Strippen des überschüssigen Phosgens 95 GC-Flächen-% o-Chlormethylbenzoylchlorid und 2,5 GC-Flächen-% nicht umgesetztes Phthalid. Die Destillation des Rohaustrages erbrachte 350 g o-Chlormethylbenzoylchlorid (93 % der Theorie) mit einer Reinheit von 97 %.

### Beispiel 2

392 g (3 mol) Phthalid wurden mit 14 g (0,15 mol) β-Picolin und 12 g (0,06 mol) BF₃-Etherat auf 140°C erhitzt. Bei 140-145°C wurden insgesamt 342 g (3,42 mol) Phosgen eingegast. Nach einer Nachreaktion von 1 h wurde das überschüssige Phosgen mit Stickstoff gestrippt. Der Reaktionsaustrag (572 g) enthielt 97 GC-Flächen-% o-Chlormethylbenzoylchlorid und 1 GC-Flächen-% Phthalid.

### Beispiel 3

Als Katalysator wurden 20 g eines zuvor hergestellten BF₃-β-Picolin-Komplexes zugegeben. Innerhalb von 6 h wurden insgesamt 230 g (2,3 mol) Phosgen eingegast. Der Reaktionsaustrag (391 g) enthielt nach dem Strippen des überschüssigen Phosgens 97,2 GC-Flächen-% o-Chlormethylbenzoylchlorid und 0,7 GC-Flächen-% Phthalid. Die Destillation des Rohaustrages erbrachte 334 g o-Chlormethylbenzoylchlorid (88 % der Theorie) mit einer Reinheit von 98,8 %.

### Beispiel 4

2 mol Phthalid (268 g) wurden mit 27,8 g Triphenylphosphinoxid (TPPO) (0,1 mol) und 1 g HY-Zeolith GE 1967 vorgelegt. Bei 140-149°C wurden innerhalb von 8 h insgesamt 231 g Phosgen (2,31 mol) eingegast. Nach einer Nachrührzeit von 1 h bei 140°C wurde das überschüssige Phosgen mit Stickstoff gestrippt. Der Austrag (403 g) enthielt 78 GC-Flächen-% o-Chlormethylbenzoylchlorid und 6,8 % Phthalid.

### Beispiel 5

2 mol Phthalid (268 g) wurden mit 27,8 g TPPO (0,1 mol) und 7,9 g (0,04 mol) BF₃-Etherat vorgelegt. Bei 140-150°C wurden innerhalb von 8 h insgesamt 233 g Phosgen (2,33 mol) eingegast. Nach einer Nachrührzeit von 1 h bei 140°C wurde das überschüssige Phosgen mit Stickstoff gestrippt. Der Austrag (415 g) enthielt 88,4 GC-Flächen-% o-Chlormethylbenzoylchlorid und 0,1 % Phthalid. Die fraktionierte Destillation des Rohaustrages lieferte 363 g (96 % d.T.) o-Chlormethylbenzoylchlorid mit einer Reinheit von 99,8 %.

### Beispiel 6

Vorgelegt wurden 268 g (2 mol) Phthalid, 9,3 g (0,1 mol) β-Picolin und 50 ml 0,1 molarer Lösung von BCl₃ in Xylol. Innerhalb von 8 h wurden bei 140-150°C insgesamt 200 g (2 mol) Phosgen eingegast. Danach ließ man eine Stunde bei 140°C nachrühren. Nach dem Strippen überschüssigen Phosgens mit Stickstoff verblieb ein Austrag von 398 g mit einem Gehalt von 77,2 GC-Flächen-% o-Chlormethylbenzoylchlorid und 10,9 % nicht umgesetzten Phthalids.

### Beispiel 7

Vorgelegt wurden 268 g (2 mol) Phthalid, 9,3 g (0,1 mol) β-Picolin und 3,1 g (0,05 mol) kristalline Borsäure (Riedel de Haën). Innerhalb von 7 h wurden bei 140-150°C insgesamt 245 g (2,45 mol) Phosgen eingegast. Danach ließ man eine Stunde bei 140°C nachrühren. Nach dem Strippen überschüssigen Phosgens mit Stickstoff verblieb ein Austrag von 380 g mit einem Gehalt von 96 GC-Flächen-% o-Chlormethylbenzoylchlorid und 1,4 % nicht umgesetzten Phthalids. Durch fraktionierte Destillation des Rohaustrages wurden 340 g o-Chlormethylbenzoylchlorid (90 % d. T.) mit einer Reinheit von > 97 % erhalten.

### Beispiel 8

Vorgelegt wurden 268 g (2 mol) Phthalid, 27,8 g (0,1 mol) TPPO und 3,1 g (0,05 mol) kristalline Borsäure (Riedel de Haën). Innerhalb von 5 h 45 min wurden bei 140-150°C insgesamt 247 g (2,47 mol) Phosgen eingegast. Danach ließ man eine Stunde bei 140°C nachrühren. Nach dem Strippen überschüssigen Phosgens mit Stickstoff verblieb ein Austrag von 411 g mit einem Gehalt von 88 GC-Flächen-% o-Chlormethylbenzoylchlorid und 0,7 % nicht umgesetzten Phthalids. Durch fraktionierte Destillation des Rohaustrages wurden 358 g o-Chlormethylbenzoylchlorid (94 % d. T.) mit einer Reinheit von 98,5 % erhalten.

### Beispiel 9

Vorgelegt wurden 268 g (2 mol) Phthalid, 27,8 g (0,1 mol) TPPO und 5,2 g (0,05 mol) Borsäuretrimethylester (Aldrich). Innerhalb von 7 h wurden bei 140-150°C insgesamt 256 g (2,56 mol) Phosgen eingegast. Danach ließ man eine Stunde bei 140°C nachrühren. Nach dem Strippen überschüssigen Phosgens mit Stickstoff verblieb ein Austrag von 410 g mit einem Gehalt von 87,6 GC-Flächen-% o-Chlormethylbenzoylchlorid und 1,1 % nicht umgesetzten Phthalids.

### Beispiel 10

Vorgelegt wurden 2 mol Phthalid, 34,8 g (0,1 mol) Cyanex 923 (Fa. Cyanamid) sowie 3,1 g (0,05 mol) Borsäure. Innerhalb von 4 h wurden bei 141 bis 150°C insgesamt 257 g (2,57 mol) Phosgen eingeleitet. Es wurde eine Stunde bei 140°C nachgerührt und das überschüssige Phosgen mit Stickstoff gestrippt. Der Rohaustrag von 417 g wurde fraktioniert destilliert. Es wurden 352 g (93% d. T.) o-Chlormethylbenzoylchlorid mit einer Reinheit von 98,7% erhalten.

### Beispiel 11

Vorgelegt wurden 2 mol Phthalid, 34,8 g (0,1 mol) Cyanex 923 (Fa. Cyanamid) sowie 5,2 g (0,05 mol) Borsäuretrimethylester. Innerhalb von 6 h wurden bei 141 bis 150°C insgesamt 244 g (2,44 mol) Phosgen eingeleitet. Es wurde eine Stunde bei 140°C nachgerührt und das überschüssige Phosgen mit Stickstoff gestrippt. Der Rohaustrag von 411 g wurde fraktioniert destilliert. Es wurden 357 g (94% d. T.) o-Chlormethylbenzoylchlorid mit einer Reinheit von 97,7% erhalten.

### Vergleichsbeispiel I gegenüber WO-A 99/16743

Gemäß der allgemeinen Versuchsvorschrift wurden 9,1 g Triethylbenzylammoniumchlorid (TEBA) (0,04 mol, 2 mol-%) und 7,9 g BF₃-Etherat (0,04 mol, 2 mol-%) als Katalysatormischung eingesetzt. Innerhalb von 11 h wurden insgesamt 45 g (0,45 mol) Phosgen bei einer Reaktionstemperatur von zunächst 95-100°C (3 h), später bei 140°C eingegast. Nach einer Nachreaktion von 1 h bei 140°C und Strippung des Phosgens enthält der Rohaustrag lediglich 7,7 GC-Flächen-% o-Chlormethylbenzoylchlorid und 91 % unumgesetztes Phthalid.

Vergleichsbeispiel I zeigt, daß unter den in WO 99/16743 bevorzugten Bedingungen die Phosgenierungsreaktion nicht durchführbar ist.

### Vergleichsbeispiel II gegenüber EP-A 583 589

2 mol Phthalid wurden mit 18,6 g β-Picolin (0,2 mol, 10 mol-%) vorgelegt. Bei 142-152°C wurden innerhalb von 10 h parallel 209 g (2,1 mol) Phosgen und 154 g HCl eingegast. Nach Nachrühren und Strippung des überschüssigen Phosgens enthielt der Reaktionsaustrag (380 g) 86 % o-Chlormethylbenzoylchlorid und 6 % unumgesetztes Phthalid.

Vergleichsbeispiel II zeigt, daß die Reaktion ohne die Zugabe einer Lewis-Säure deutlich langsamer und mit schlechterem Umsatz abläuft und ferner eine höhere Menge des Phosgenierungskatalysators erforderlich ist.

## Patentansprüche

1. Verfahren zur Herstellung von o-Chlormethylbenzoesäurechloriden der Formel I, 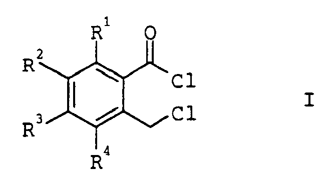 in der R¹ bis R⁴ gleich oder verschieden sein können und für Wasserstoff, C₁-C₄-Alkyl, Halogen oder Trifluormethyl stehen, durch Umsetzung von benzokondensierten Lactonen der Formel II, 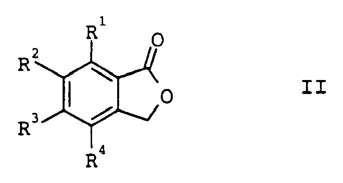 in der R¹ bis R⁴ die vorgenannte Bedeutung besitzen, mit gasförmigem oder flüssigem Phosgen, dessen Dimeren oder Trimeren,
**dadurch gekennzeichnet, daß** man die Umsetzung in Gegenwart katalytischer Mengen einer Lewis-Säure und katalytischer Mengen eines Phosgenierungskatalysators durchführt.

2. Verfahren gemäß Anspruch 1, **dadurch gekennzeichnet, daß** als Lewis-Säure eine Borverbindung eingesetzt wird.

3. Verfahren gemäß Anspruch 2, **dadurch gekennzeichnet, daß** als Lewis-Säure Bortrifluorid oder Bortrichlorid in Komplex-gebundener Form eingesetzt wird.

4. Verfahren gemäß Anspruch 2, **dadurch gekennzeichnet, daß** als Lewis-Säure Borsäure oder ein Borsäuretri-C₁-C₄-alkylester eingesetzt wird.

5. Verfahren gemäß Anspruch 1, **dadurch gekennzeichnet, daß** als Lewis-Säure Aluminiumsilikate vom Zeolith-Typ verwendet werden.

6. Verfahren gemäß einem der Ansprüche 1 bis 5, **dadurch gekennzeichnet, daß** die Lewis-Säure in einer Konzentration von 0,1 bis 20 mol-% bezogen auf das Lacton II eingesetzt wird.

7. Verfahren gemäß einem der Ansprüche 1 bis 3 **dadurch gekennzeichnet, daß** als Katalysator 0,1 bis 20 mol-% eines Komplexes aus Lewis-Säure und Phosgenierungskatalysator eingesetzt werden.

8. Verfahren gemäß einem der Ansprüche 1 bis 6, **dadurch gekennzeichnet, daß** als Phosgenierungskatalysator ein Pyridin der Formel IIIa oder ein Phospin(oxid) der Formel IIIb, 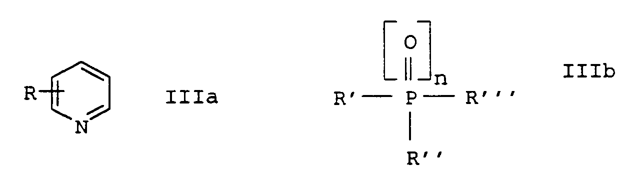 wobei in Formel IIIa R für Wasserstoff oder Methyl steht, und in Formel IIIb R' bis R"' gleich oder verschieden sein können und C₁-C₁₀-Alkyl oder unsubstituiertes oder durch C₁-C₄-Alkyl substituiertes Phenyl bedeuten und der Index n für 0 oder 1 steht, eingesetzt wird.

9. Verfahren nach Anspruch 8, **dadurch gekennzeichnet, daß** als Phosgenierungskatalysator 3-Methylpyridin (β-Picolin), Triphenylphosphinoxid oder ein flüssiges Trialkylphosphinoxid verwendet wird.

10. Verfahren gemäß einem der Ansprüche 1 bis 8, **dadurch gekennzeichnet, daß** 0,1 bis 20 mol-% des Phosgenierungskatalysators bezogen auf das Lacton II eingesetzt werden.

## Claims

1. A process for preparing o-chloromethylbenzoyl chlorides of the formula I, 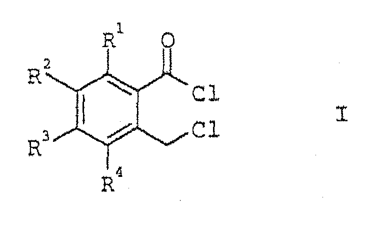 in which R¹ to R⁴ can be identical or different and are hydrogen, C₁-C₄-alkyl, halogen or trifluoromethyl, by reacting benzo-fused lactones of the formula II, 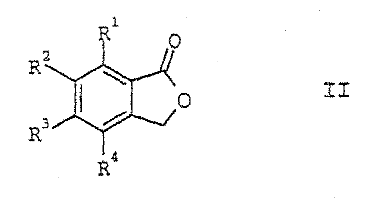 in which R¹ to R⁴ are as defined above, with gaseous or liquid phosgene, its dimers or trimers,
which comprises carrying out the reaction in the presence of catalytic amounts of a Lewis acid and catalytic amounts of a phosgenation catalyst.

2. The process according to claim 1, wherein the Lewis acid used is a boron compound.

3. The process according to claim 2, wherein the Lewis acid used is boron trifluoride or boron trichloride in coordinate form.

4. The process according to claim 2, wherein the Lewis acid used is boric acid or a tri-C₁-C₄-alkyl borate.

5. The process according to claim 1, wherein the Lewis acid used is an alumosilicate of the zeolite type.

6. The process according to any of claims 1 to 5, wherein the Lewis acid is employed in a concentration of from 0.1 to 20 mol%, based on the lactone II.

7. The process according to any of claims 1 to 3, wherein the catalyst used is from 0.1 to 20 mol% of a complex of Lewis acid and phosgenation catalyst.

8. The process according to any of claims 1 to 6, wherein the phosgenation catalyst used is a pyridine of the formula IIIa or a phosphine (oxide) of the formula IIIb, 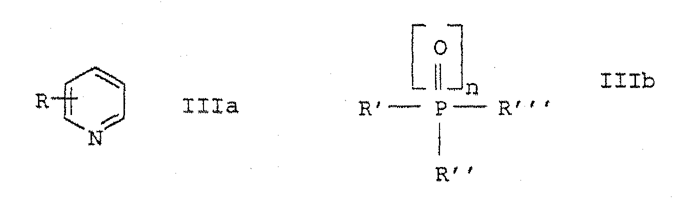 where in the formula IIIa, R is hydrogen or methyl, and in the formula IIIb, R' to R"' can be identical or different and are C₁-C₁₀-alkyl or unsubstituted or C₁-C₄-alkyl-substituted phenyl and the index n is 0 or 1.

9. The process according to claim 8, wherein the phosgenation catalyst used is 3-methylpyridine (β-picoline), triphenylphosphine oxide or a liquid trialkylphosphine oxide.

10. The process according to any of claims 1 to 8, wherein from 0.1 to 20 mol% of the phosgenation catalyst, based on the lactone II, are used.

## Revendications

1. Procédé pour la production de chlorures d'acides o-chlorométhylbenzoïques de formule I, 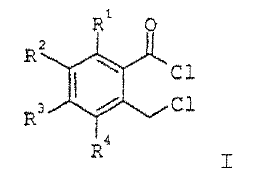 dans laquelle R¹ à R⁴ peuvent être identiques ou différents et représentent un atome d'hydrogène ou d'halogène, un groupe alkyle en C₁-C₄ ou trifluorométhyle, par mise en réaction de lactones condensées à un noyau benzénique, de formule II 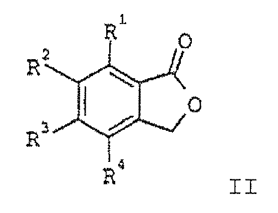 dans laquelle R¹ à R⁴ ont les significations données ci-dessus, avec du phosgène liquide ou gazeux, son dimère ou trimère,
**caractérisé en ce qu'**on effectue la réaction en présence de quantités catalytiques d'un acide de Lewis et de quantités catalytiques d'un catalyseur de phosgénation.

2. Procédé selon la revendication 1, **caractérisé en ce qu'**on utilise comme acide de Lewis un composé contenant du bore.

3. Procédé selon la revendication 2, **caractérisé en ce qu'**on utilise comme acide de Lewis le trifluorure de bore ou le trichlorure de bore sous forme liée en complexe.

4. Procédé selon la revendication 2, **caractérisé en ce qu'**on utilise comme acide de Lewis l'acide borique ou un borate d'alkyle en C₁-C₄.

5. Procédé selon la revendication 1, **caractérisé en ce qu'**on utilise comme acide de Lewis des silicates d'aluminium du type zéolithe.

6. Procédé selon l'une quelconque des revendications 1 à 5, **caractérisé en ce qu'**on utilise l'acide de Lewis à une concentration de 0,1 à 20 % en moles, par rapport à la lactone II.

7. Procédé selon l'une quelconque des revendications 1 à 3, **caractérisé en ce qu'**on utilise comme catalyseur de 0,1 à 20 % en moles d'un complexe d'acide de Lewis et catalyseur de phosgénation.

8. Procédé selon l'une quelconque des revendications 1 à 6, **caractérisé en ce qu'**on utilise comme catalyseur de phosgénation une pyridine de formule IIIa ou un(e) (oxyde de) phosphine de formule IIIb, 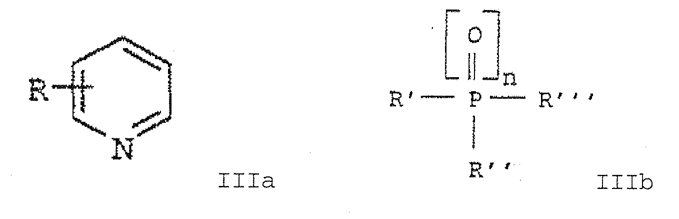 dans la formule IIIa R représentant un atome d'hydrogène ou le groupe méthyle, et dans la formule IIIb R' à R"' pouvant être identiques ou différents et représentant un groupe alkyle en C₁-C₁₀ ou un groupe phényle non substitué ou substitué par alkyle en C₁-C₄ et l'indice n représentant 0 ou 1.

9. Procédé selon la revendication 8, **caractérisé en ce qu'**on utilise comme catalyseur de phosgénation la 3-méthylpyridine (β-picoline), l'oxyde de triphénylphosphine ou un oxyde de trialkylphosphine liquide.

10. Procédé selon l'une quelconque des revendications 1 à 8, **caractérisé en ce qu'**on utilise de 0,1 à 20 % en moles du catalyseur de phosgénation, par rapport à la lactone II.
